(19) Europäisches Patentamt
European Patent Office
Office européen des brevets

(11)  **EP 2 698 110 A1**

(12)  **EUROPEAN PATENT APPLICATION**

(43) Date of publication:
**19.02.2014 Bulletin 2014/08**

(51) Int Cl.:
***A61B 5/113*** *(2006.01)*

(21) Application number: **13190157.1**

(22) Date of filing: **26.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.02.2009 US 155992 P**
**27.02.2009 US 155997 P**
**27.02.2009 US 156104 P**

(62) Document number(s) of the earlier application(s) in accordance with Art. 76 EPC:
**10706116.0 / 2 323 552**

(71) Applicant: **Pneumosonics, Inc.**
**Cleveland, OH 44114 (US)**

(72) Inventors:
• **Fell, Roger, B.**
**AVON LAKE, OH Ohio 44012 (US)**

• **Wilder, Steve**
**ASHLAND, OH Ohio 44805 (US)**
• **Theobold, David**
**CHAPEL HILL, NC North Carolina 27516-9305 (US)**
• **Yoder, Raymond, Lloyd**
**WILLOUGHBY, OH Ohio 44094 (US)**

(74) Representative: **Ilgart, Jean-Christophe**
**BREVALEX**
**95 rue d'Amsterdam**
**75378 Paris Cedex 8 (FR)**

Remarks:
This application was filed on 24-10-2013 as a divisional application to the application mentioned under INID code 62.

(54)  **Non-invasive pneumothorax detection method and apparatus**

(57)     Provided is a micropower impulse radar (MIR) pneumothorax detector. The detector includes a processor and a handheld MIR scanner. The handheld MIR scanner includes a device housing, an antenna located within the device housing, circuitry that generates MIR signals, and circuitry that processes received echoes of the MIR signals and provides the processed received echoes to the processor. The processor automatically determines whether a pneumothorax is present based on the processed received echoes of the MIR signals.

EP 2 698 110 A1

**Description**

CROSS-REFERENCE TO RELATED APPLICATIONS

Benefit of U.S. Provisional Patent Applications Serial Nos. 61/155,992, 61/155,997, and 61/156,104 filed on February 27, 2009, is hereby claimed and the disclosures of these applications are incorporated herein by reference in their entireties.

BACKGROUND OF THE INVENTION

Field of the Invention

The present invention is related to devices and methods for detecting a pneumothorax, and more specifically to the automatic detection of a pneumothorax.

Description of Related Art

Pneumothorax is a medical condition in which a pocket of air is trapped in the pleural space around the lungs, making breathing more difficult. In some cases this can lead to a collapse of the lungs and possibly even death. It is most often caused by blunt trauma to the chest, such as the trauma experienced in some car accidents. Pneumothorax can be diagnosed using x-ray or ultrasound imaging systems commonly found in hospitals.

BRIEF SUMMARY OF THE INVENTION

One problem associated with diagnosing a pneumothorax is that the x-ray or ultrasound imaging systems used in the diagnosis are either not portable or not readily portable, and may require a large display for displaying images. The patient may have to be brought to the equipment, such as to a hospital, before a diagnosis can occur. Furthermore, interpretation of images by a skilled person, such as a doctor, is required to diagnose the pneumothorax. It would be beneficial to provide a highly portable device, such as a handheld device, that is capable of automatically determining the presence of a pneumothorax. Such a device could be carried and used by  minimally trained operators, such as emergency medical technicians and battlefield medics, as part of a standard equipment package for use as needed.
In accordance with one aspect of the present invention, provided is a micropower impulse radar (MIR) pneumothorax detector. The detector includes a processor and a handheld MIR scanner. The handheld MIR scanner includes a device housing, an antenna located within the device housing, circuitry that generates MIR signals, and circuitry that processes received echoes of the MIR signals and provides the processed received echoes to the processor. The processor automatically determines whether a pneumothorax is present based on the processed received echoes of the MIR signals.
In accordance with another aspect of the present invention, provided is a method of deriving pneumothorax information from a body using micropower impulse radar (MIR) signals comprising subjecting the body to MIR signals. A handheld MIR scanning device is provided. The MIR scanning device transmits MIR signals and receives and processes echoes of the MIR signals. A MIR signal is sent into a first right torso location of the body and a reflected MIR signal is received using the MIR scanning device. A MIR signal is sent into a second right torso location of the body and a reflected MIR signal is received using the MIR scanning device. A MIR signal is sent into a first left torso location of the body and a reflected MIR signal is received using the MIR scanning device. A MIR signal is sent into a second left torso location of the body and a reflected MIR signal is received using the MIR scanning device. At least one of the reflected MIR signals is compared to reference data. An output is generated based on a result of the step of comparing at least one of the reflected MIR signals to the reference data.

BRIEF DESCRIPTION OF THE DRAWINGS

FIG. 1 is a perspective view of a pneumothorax detector;
FIG. 2 is a perspective view of the pneumothorax detector;
FIG. 3 is an exploded view of the pneumothorax detector;
FIG. 5 shows a human torso;
FIG. 6 shows a human torso;
FIG. 7 shows a human torso;

FIG. 8 shows a human torso;
FIG. 9 is a flowchart;
FIG. 10 is a graph of obtained data;
FIG. 11 is a correlation chart;
FIG. 12 is a graph of obtained data;
FIG. 13 is a graph of simulated data;
FIG. 14 is a block diagram of MIR circuitry;
FIG. 15 is a block diagram of MIR circuitry;
FIG. 16 is a partial schematic diagram of MIR circuitry;
FIG. 17 is a partial schematic diagram of MIR circuitry;
FIG. 18 is a partial schematic diagram of MIR circuitry;
FIG. 19 is a partial schematic diagram of MIR circuitry;
FIG. 20 is a partial schematic diagram of MIR circuitry;
FIG. 21 is a partial schematic diagram of MIR circuitry;
FIG. 22 is a partial schematic diagram of MIR circuitry;
FIG. 23 is a partial schematic diagram of MIR circuitry;
FIG. 24 is a partial schematic diagram of MIR circuitry;
FIG. 25 is a partial schematic diagram of MIR circuitry;
FIG. 26 is a partial schematic diagram of MIR circuitry;
FIG. 27 is a partial schematic diagram of MIR circuitry;
FIG. 28 is a partial schematic diagram of MIR circuitry;
FIG. 29 is a perspective view of an antenna;
FIG. 30 is a perspective view of the antenna; and
FIG. 31 is a perspective view of the antenna.

DETAILED DESCRIPTION OF THE INVENTION

The present invention is related to the automatic detection of a pneumothorax. The present invention will now be described with reference to the drawings, wherein like reference numerals are used to refer to like elements throughout. It is to be appreciated that the various drawings are not necessarily drawn to scale from one figure to another nor inside a given figure, and in particular that the size of the components are arbitrarily drawn for facilitating the understanding of the drawings. In the following description, for purposes of explanation, numerous specific details are set forth in order to provide a thorough understanding of the present invention. It may be evident, however, that the present invention can be practiced without these specific details. Additionally, other embodiments of the invention are possible and the invention is capable of being practiced and carried out in ways other than as described. The terminology and phraseology used in describing the invention is employed for the purpose of promoting an understanding of the invention and should not be taken as limiting.

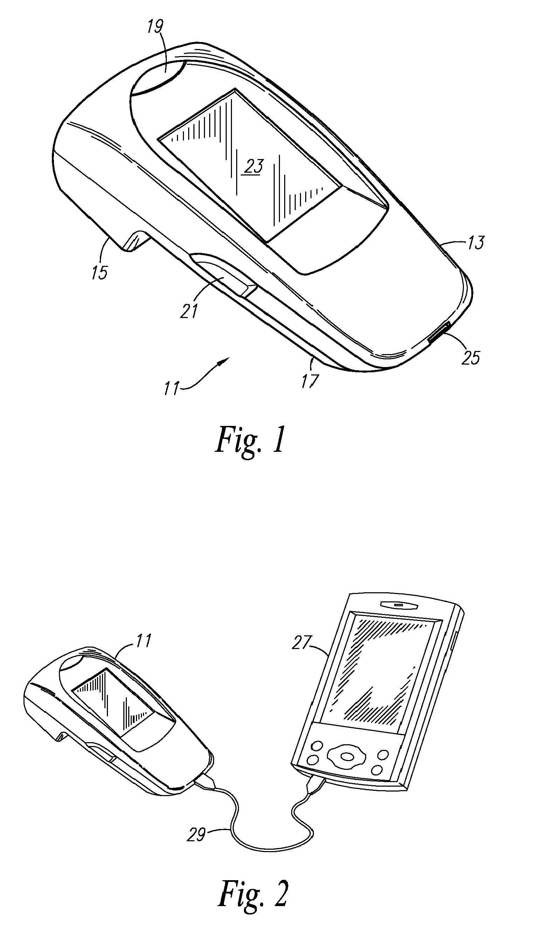

FIG. 1 is a perspective view of a handheld, non-invasive pneumothorax detector 11 to assist in the detection of a suspected pneumothorax. Using an extremely low power radar technology known as micropower impulse radar (MIR) to obtain data about a patient, the pneumothorax detector 11 process the data to automatically determine the presence of a pneumothorax. As will be described in detail below, the pneumothorax detector 11 is used to scan several different locations on the patient's body to determine the presence of a pneumothorax.

The pneumothorax detector 11 includes an outer housing 13 having a patient contact area 15 that is placed onto the patient, and a grip area 17 that is held by an operator. The pneumothorax detector 11 includes a power button 19 for turning the device on and off, a left scan button 21 and a right scan button (not shown). The left 21 and right scan buttons operate identically and, when activated, cause the pneumothorax detector 11 to initiate a scan operation. A scan operation can be executed based on a momentary activation of a scan button, or the pneumothorax detector can require that the activation of the scan button be maintained for the entire scan operation. A speaker (not shown) within the pneumothorax detector 11 can provide an audible indication that a scan operation is complete, that a pneumothorax has been detected, etc. The pneumothorax detector 11 includes an optional display 23, such as a liquid crystal display (LCD), to provide information to the operator such as instructions regarding the scanning process, absence or presence of a pneumothorax, scanning errors, battery status, etc. The pneumothorax detector 11 can further include indicator LEDs to provide said and/or other information.

The left 21 and right scan buttons provide for convenient operation of the pneumothorax detector 11 in either the left or right hand of an operator. Information, such as patient identification information, can be entered using the scan buttons. The display 23 can be a touch screen display, and information can be entered using the touch screen.

The pneumothorax detector 11 includes one or more universal serial bus (USB) ports 25. A battery for powering the pneumothorax detector 11 can be charged through the USB port 25, or the pneumothorax detector can be directly powered through USB port. Also, data, such processed received echoes of MIR signals, patient status, etc., can be transferred from the pneumothorax detector to a remote processor through the USB port 25. An example remote processor is shown in FIG. 2, wherein the pneumothorax detector 11 is connected to a remote personal digital assistant (PDA) 27 through the USB port 25 over a cable 29. It is to be appreciated that a wireless data connection could be provided between the pneumothorax detector 11 and the PDA 27. The data processing functions or algorithm for determining a pneumothorax (described below) can be performed by the pneumothorax detector 11 or the PDA 27. In addition to the PDA, other example remote processors include laptop, notebook and tablet computers, desktop computers, and the like. In an embodiment, the remote processor is a MOTOROLA MC7x (e.g., MC75) using WINDOWS MOBILE 6.x.

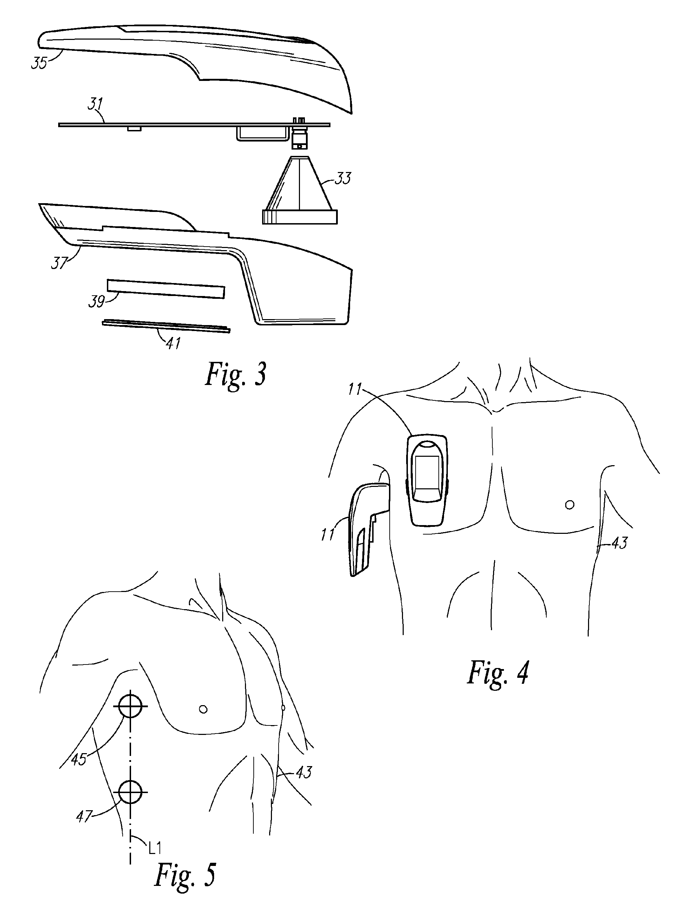

FIG. 3 is an exploded view of the pneumothorax detector 11. A circuit board or MIR board 31 is operatively connected to an antenna 33, and the MIR board 31 and antenna 33 are located within upper 35 and lower 37 housing members. The pneumothorax detector 11 further includes a battery 39 and a battery cover 41.

The antenna 33 is shown as a horn antenna. However, the antenna can be any type of antenna suitable for use with MIR. Example antennas include the resonant monopole, log-periodic toothed trapezoid antenna (LPTTA), cavity-backed Archemedian spiral (CBAS), Vivaldi antenna, and Bi-conical antenna.

With reference to FIGS. 4-9, a patient scanning procedure for automatically determining the presence of a pneumothorax will be described. A plurality of locations on the right and/or left sides of the patient's torso are scanned in sequence, and the results of the scanning are processed by the pneumothorax detector or the remote processor to determine whether the patient has a pneumothorax. Scanning involves sending MIR signals (e.g., pulses) into the patient's torso and receiving reflections of the MIR signals from the patient's body. In an embodiment, the patient contact area 15 of the pneumothorax detector 11 is placed into direct contact with the patient's skin during scanning.

The patient can be supine, upright or partially reclined during scanning, and the pneumothorax detector can be oriented in any direction relative to the patient's torso. However, for best results, the patient should be supine during scanning and the pneumothorax detector 11 oriented so that its longitudinal axis is generally parallel to the anterior axillary line and/or the midclavicular line of the patient's torso 43 (see FIG. 4).

As shown in FIGS. 5-8, a plurality of locations (e.g., two locations, three locations, four locations, eight locations, more than eight locations, etc.) on the right and/or left sides of the torso 43 are scanned in sequence, and the results of the scanning are processed to determine the presence of a pneumothorax. In an embodiment, four locations on the right side of the torso 43 are scanned first, followed by the scanning of four locations on the left side of the torso 43. For each side, two of the four locations are located substantially on the anterior axillary line L1, L3 and two of the four locations are located substantially on the midclavicular line L2, L4.

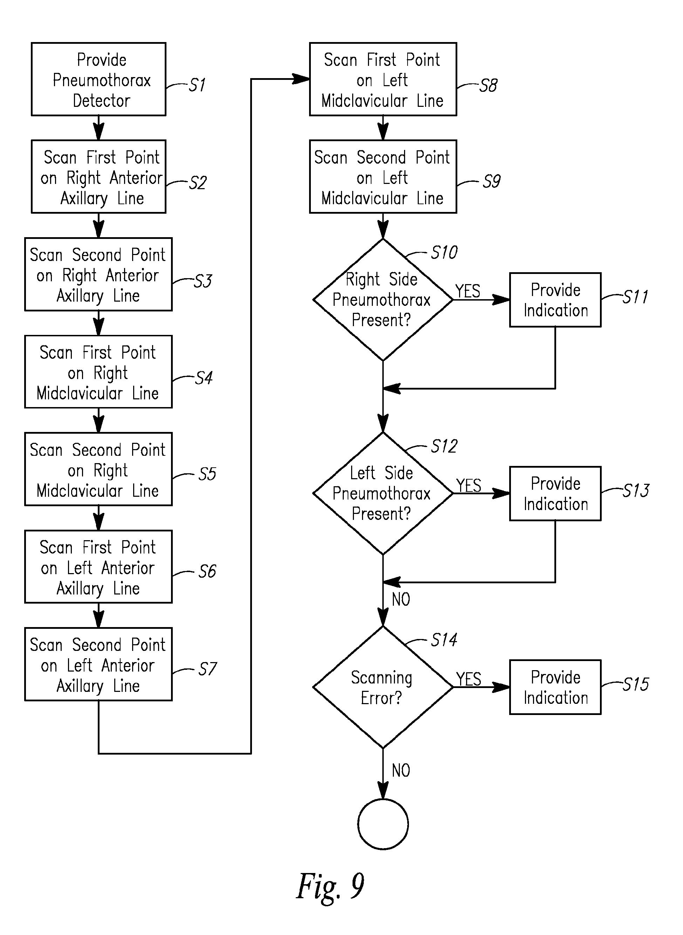

A flowchart of an example scanning process is provided in FIG. 9 and will be discussed in conjunction with FIGS. 5-8. The pneumothorax detector is provided in step S1. Using the pneumothorax detector, the 4th rib interspace (just above the 5th rib) on the right anterior axillary line L1 (point 45) can be scanned first (step S2), followed by the 6th rib interspace (just above the 7th rib) on the right anterior axillary line (point 47) (step S3). Then the 2nd rib interspace (just above the 3rd rib) on the right midclavicular line L2 (point 49) can be scanned (step S4), followed by the 5th rib interspace (just above the 6th rib) on the right midclavicular line (point 51) (step S5). Then the 4th rib interspace on the left anterior axillary line L3 (point 53) can be scanned (step S6), followed by the 6th rib interspace on the left anterior axillary line (point 55) (step S7). Then the 2nd rib interspace on the left midclavicular line L4 (point 57) can be scanned (step S8), followed by the 5th rib interspace on the left midclavicular line (point 59) (step S9). The results of scanning the right side of the torso are analyzed to determine whether a right-side pneumothorax is present (step S10). If a right-side pneumothorax is present, then an appropriate indication is provided to the operator, such as via the display and/or an LED (step S11). The results of scanning the left side of the torso are analyzed to determine whether a left-side pneumothorax is present (step S12). If a left-side pneumothorax is present, then an appropriate indication is provided to the operator (step S13). The results of scanning the right and left sides of the torso can be analyzed to determine whether a scanning error has occurred (step S14). For example, the presence of metal in the torso (e.g., shrapnel) can cause a scanning error, or the scanning of the air rather than the torso directly can cause a scanning error. If a scanning error occurs, then an appropriate indication is provided to the operator (step S15). It is to be appreciated that various scanning locations and sequences are possible. Examples of the analysis that can be performed in steps S10 and S14 are discussed further below.

The pneumothorax detector 11 operates by sending an MIR pulse into the torso of the patient and then receiving a reflection or echo from the patient. The signal processing of pneumothorax detector 11 is timed to process reflections so that the reflections correspond to the proper depth for a pneumothorax, if one was present. Due to the presence of air in the pleural cavity, more reflection of the MIR pulse is observed when a pneumothorax is present. By analyzing the reflections at the proper time (i.e., reflections from the proper depth), the presence of a pneumothorax can be determined.

One method for determining the presence of a pneumothorax includes capturing multiple reflected waveforms for each scanning location. The multiple reflected waveforms can be averaged to obtain an average waveform for each scanning location. The average waveforms can then be compared to reference waveforms (e.g., "normal" waveforms) to determine whether a pneumothorax is present. For example, a standard deviation can be calculated for each scanning location using the average and normal waveforms. Mean standard deviations for the right and left sides of the torso can be respectively calculated from the standard deviations corresponding to the right side scanning locations and the standard deviations corresponding to the left side scanning locations, to determine a right side pneumo index $\sigma_R$ and a left side pneumo index $\sigma_L$. That is, the right side pneumo index $\sigma_R$ is the mean of the standard deviations for the right side scanning locations, and the left side pneumo index $\sigma_L$ is the mean of the standard deviations for the left side scanning locations. The pneumo indices $\sigma_R$, $\sigma_L$ can be compared to threshold values to determine whether a pneumothorax is present or whether a scanning error occurred. For example, if a pneumo index is less than a normal-to-pneumo threshold, then the corresponding side does not have a pneumothorax. If the pneumo index is greater than the normal-to-pneumo threshold but less than a pneumo-to-error threshold, then a pneumothorax is present in the corresponding side. If the pneumo index is greater than the pneumo-to-error threshold, then a scanning error has occurred with respect to the corresponding side.

Another method for determining the presence of a pneumothorax includes capturing multiple frames of data (e.g., 5 frames) for each scanned chest location (e.g., points 45-59 in FIGS. 5-8). Each frame can have a number of 16-bit integer samples (e.g., 470) provided by an A/D converter.

Therefore, a frame from the A/D typically consists of 470 points, roughly corresponding to time from the electronics, through the cable, antenna, and then into the body. Since any return from the electronics and antenna conveys no useful information and can desensitize the detection process, irrelevant samples should be removed from the frame. This can be done through windowing the frame at the front of the frame to improve pneumothorax detection. There is currently no reason to truncate any points off the end of the frame, since relevant data may be present up to the end of the frame, depending upon the setting of the electronics in the detector. For each frame, whether a reference frame used for comparison purposes or a data frame, a fixed number of points from the beginning of the frame are eliminated. The piece to eliminate will be referred to as the "precursor" and its length will be ascertained in a calibration procedure, described later.

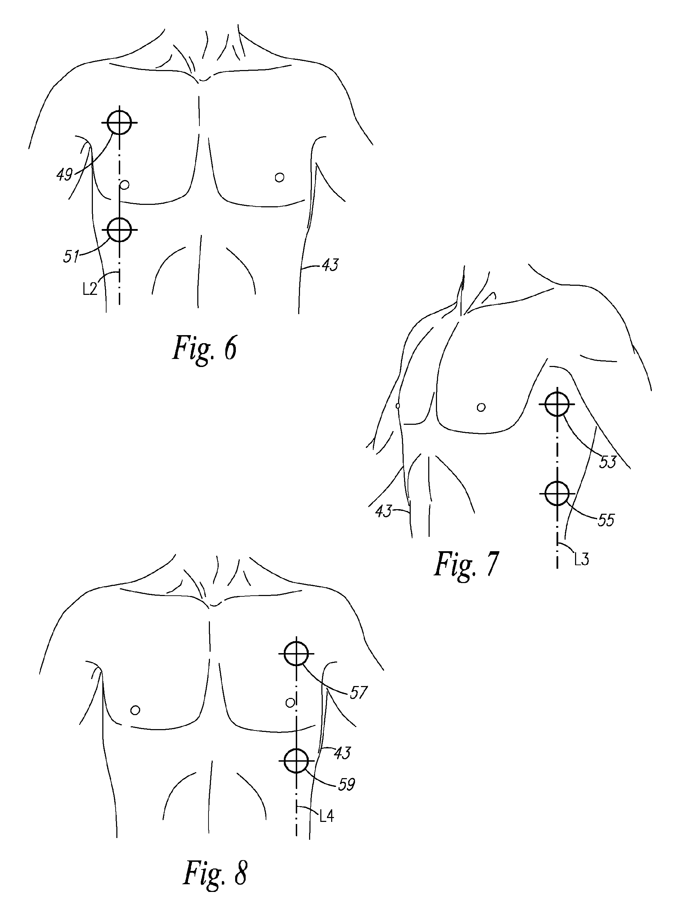

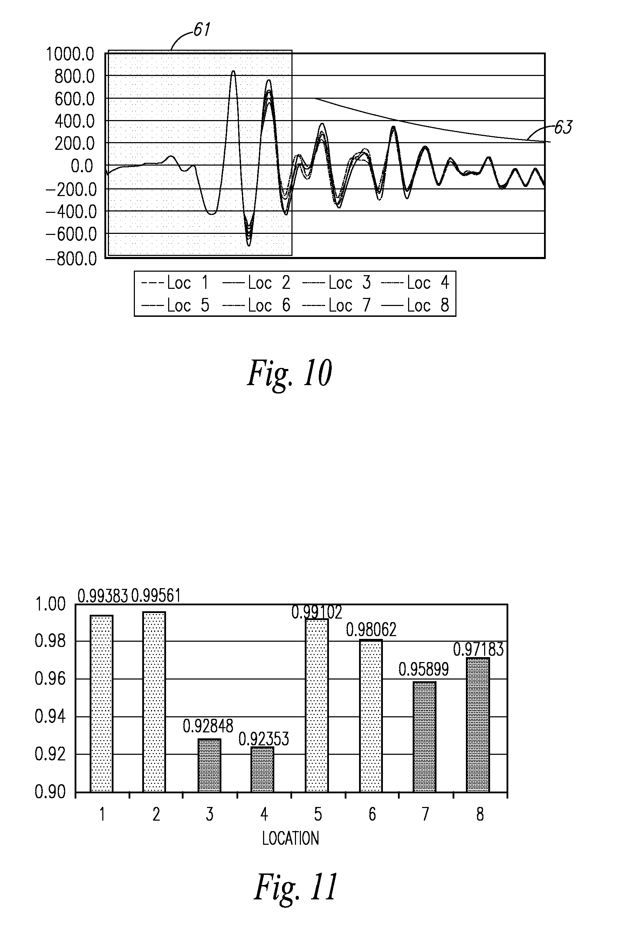

Frame Scaling: Turning to FIG. 10, the boxed area 61 represents that portion of the precursor data that will be windowed (eliminated) from the processing. Loc 1 through Loc 8 refers to the eight scanned chest locations with respect to a given frame (e.g., frame 1 for each location). Loc 1 corresponds to point 45 in FIG. 5. Loc 2 corresponds to point 47 in FIG. 5. Loc 3 corresponds to point 49 in FIG. 6. LOC 4 corresponds to point 51 in FIG. 6. Loc 5 corresponds to point 53 in FIG. 7. Loc 6 corresponds to point 55 in FIG. 7. Loc 7 corresponds to point 57 in FIG. 8. Loc 8 corresponds to point 59 in FIG. 8. Hereinafter, reference to locations 1-8 refer to points 45-59 from FIGS. 5-8, respectively.

A universal property of the MIR data is that after the precursor, the return amplitudes decay in a somewhat exponential fashion, as indicated by the curved line 63 in FIG. 10. Such a decay characteristic is due to losses incurred while traversing the propagation medium or electronics decreasing in an exponential fashion. Hence, if the decay function is F(t), multiplying by 1 / F(t) from the end of the precursor to the end of the frame will restore the "importance" of the data in the latter part of the frame. Since the raw data is monopolar (positive values), the frame is made equally variable about zero in order to do multiplicative scaling. Then, a scaling can be applied to eliminate the decay characteristic. The scaling need only be applied to the data after the precursor. The goal is to start with the "good" data and begin with a weighting of one; then progressively amplify by higher and higher values as the frame progresses, in order to equalize the return.

First, window the frame as described above. Second, find the average of the remaining data. Third, multiply the remaining data minus the average by a function G(t) ~ 1 / F(t) where F(t) is the decay function and t is the sample number minus the length of the precursor (that is, we want the retained data to begin with sample number zero). This function will be of the form: G(t) = a • exp(k • t). The values of amplitude factor a and decay factor k are obtained from a calibration procedure, described later. The values of these coefficients are not expected to vary for any particular device. Once a device is calibrated, only minor changes are expected to occur from component drift. Note: As a matter of simplification, let a = 1, i.e. so that the amplitude of G(t) will equal one when t = 0 (at the beginning of the good data).

Reference Data: Whatever processing function is used to determine the presence of a pneumothorax (e.g., variance, correlation, pattern matching, wavelets, etc.); a comparison is made in order to make a Yes/No decision on the presence of a pneumothorax. In other words, a reference frame is needed. First, it should represent the hardware being used. In that role, it eliminates device-to-device variation. If equipment drift is experienced, the reference frame needs to be reestablished. Second, it should be suitable for making the required comparisons independent of the patient being examined (e.g., man, woman, thin, portly, etc.). With that in mind, self-referencing is a solution - the reference frame should come from the patient data. By doing so, the first condition is met as well (equipment drift is eliminated if a separate reference frame is obtained from each patient).

As a first step, establish a set of default reference candidates. Use the most "benign" positions from which to build the reference.

Approach A: Average the frame data from locations {1,2,5,6}. The frames from each of these locations {1,2,5,6} would have been windowed, scaled, and have zero mean. Hence, after they are averaged (producing the reference vector), the reference vector will be zero mean as well (the average of the means is identical to the mean of the average).

Once the reference vector is scaled, its standard deviation should be found, since it is needed for calculations used in the detection process (discussed below).

Approach B: There is no guarantee that this default constructor is best for all scans. For example, there may be contamination of one or more of the reference locations {1,2,5,6} by a pneumothorax or other anomaly in the chest. Since there is no fundamental reference with which to compare (that, after all, is what is being determined), the next best thing is to do a cross-correlation between pairs of the four candidate locations {1,2,5,6} to see if they are similar.

The correlation function is: $\rho_{A,B} = \dfrac{\dfrac{1}{n}\sum_{1}^{n}(a_i \bullet b_i)}{\sigma_A \bullet \sigma_B}$ , where n are the points following the precursor to the end of the frame, A is data from one location, B is data from another, and $\sigma_A$ and $\sigma_B$ are the standard deviations of A and B. Both A and B are assumed to be zero mean.

An algorithm that eliminates contamination of a single bad reference location is:

Establish a reference threshold, $T_r$, with a large correlation, say 0.80 or greater
Set the reference to {}, i.e. No Reference

$$\text{IF } ( \rho_{1,5} > T_r \cap \rho_{2,6} > T_r \cap \rho_{1,2} > T_r \cap \rho_{5,6} > T_r ) \text{ THEN } \{1,2,5,6\}$$

$$\text{ELSE}$$

$$\text{IF } ( \rho_{1,2} \le T_r \cup \rho_{5,6} \le T_r ) \text{ THEN}$$

$$\text{IF } (\rho_{1,5} > T_r ) \text{ THEN } \{1,5\}$$

$$\text{ELSE}$$

$$\text{IF } (\rho_{2,6} > T_r ) \text{ THEN } \{2,6\}$$

$$\text{ENDIF}$$

$$\text{ENDIF}$$

This methodology will eliminate using reference data from a side pair or lateral pair that is contaminated (not similar). This results in one of the two lateral pairs being used as the reference. If no reference is suitable, the algorithm drops through with "No Reference", meaning bad scan.

Approach C: The previous approach works well if there is lateral or vertical similarity in the highly correlated pairs. However, it is possible that highly correlated pairs can be contained in any geometry (lateral, vertical, diagonal, or in the limit, between any data locations on the chest (without pneumothorax). A relatively straightforward algorithm can be devised to build a reference for such a case. First, select a set of candidate locations, say {1,2,5,6}, as before. Next, produce all correlation pairs {$\rho_{1,2}\ \rho_{1,5}\ \rho_{1,6}\ \rho_{2,5}\ \rho_{2,6}\ \rho_{5,6}$}. Hence, we have all vertical, horizontal, and diagonal pairs. This methodology can be extended to any number of locations on the chest (e.g., up to 8), producing all non-repeating combinations of two. Set a threshold for the correlation that will define "similarity". A good candidate is $\rho > 0.80$. Compare the sets of $\rho$ to the threshold and if they meet or exceed that number, build a vector of length eight (the number of locations), keeping track of how many times a location shows up in a correlation pair exhibiting high similarity.

For example, locations {1,2,5,6} are candidates for the reference. Location 2 is not similar to 1, 5, or 6 for some reason. Hence, the pairs having high correlation will be:

$$\{ \rho_{1,5}\ \ \rho_{5,6}\ \ \rho_{1,6} \}.$$

Build a vector corresponding to locations 1,2,3,4,5,6,7,8 that counts the number of times that a location showed up in the highly correlated pairs, i.e., 1,5,5,6,1,6 (tally the correlation subscripts) :

$$\{2\ 0\ 0\ 0\ 2\ 2\}$$

Pick a minimum number of times that a location must occur within this vector for it to be deemed a suitable location to contribute to the reference. A workable number is 2. Hence, locations 1, 5, and 6 are acceptable reference candidates. Average these three locations for each point in time, thereby producing a highly correlated reference vector {1, 5, 6}. This methodology can be extended to any or all of the eight locations. In fact, if one or more locations are included more than once (say, use 1 and 5 twice for {1,1,2,5,5,6} ), then their contributions will be multiplied by the number of times that they are used.

Pneumothorax Detection: Once a reference vector is established, the presence of a pneumothorax or absence can be decided from the four frames gathered at the front of the chest (location 3/point 49, location 4/point 51, location 7/point 57, and location 8/point 59). Bilateral pneumothorax may be present, but the goal is to simply identify the presence, independent of location. Pneumothorax presents itself in the MIR device as a disturbance in the waveform that occurs after the precursor. It is evidenced by an amplitude change. Phase (zero-crossing) changes are very minor in nature and need not be considered. Since scaling removes the mean and "restores" any decay distortion, the simple statistical procedure of correlation to the reference vector will identify pneumothorax induced amplitude changes with a high probability.

In order to self-calibrate the detection process, remove patient-to-patient (or for that matter, location-to-location) measurement dependence. Pearson correlation accomplishes that function since the resultant correlation coefficient is normalized by the standard deviation of the two vectors being compared. As an example, FIG. 11 displays Pearson correlation coefficients for a patient with a right side pneumothorax (locations 3 and 4). The bars for locations 1, 2, 5 and 6 (which can be used to establish the reference vector, but need not be used in the MIR detection processing itself) are presented for reference. These locations (also correlated against the reference vector) are all above 0.98 (they are 98% similar to the reference vector), clearly showing that the side locations are isolated from any pneumothorax influence. The bars for locations 3, 4, 7 and 8 are the front chest location correlations, of which locations 3 and 4 (on the right side of the chest) have less similarity to the reference vector. The correlation function used is:

$$\rho_{A,B} = \frac{\frac{1}{n}\sum_{1}^{n}(a_i \bullet b_i)}{\sigma_A \bullet \sigma_B}$$

where n are the points following the precursor to the end of the frame, A is the reference vector, B is the location data, and $\sigma_A$ and $\sigma_B$ are the standard deviations of A and B. Both A and B are assumed to be zero mean. If a threshold is established, say 0.90, then a pneumothorax would be identified if any of the four locations fell below that threshold.

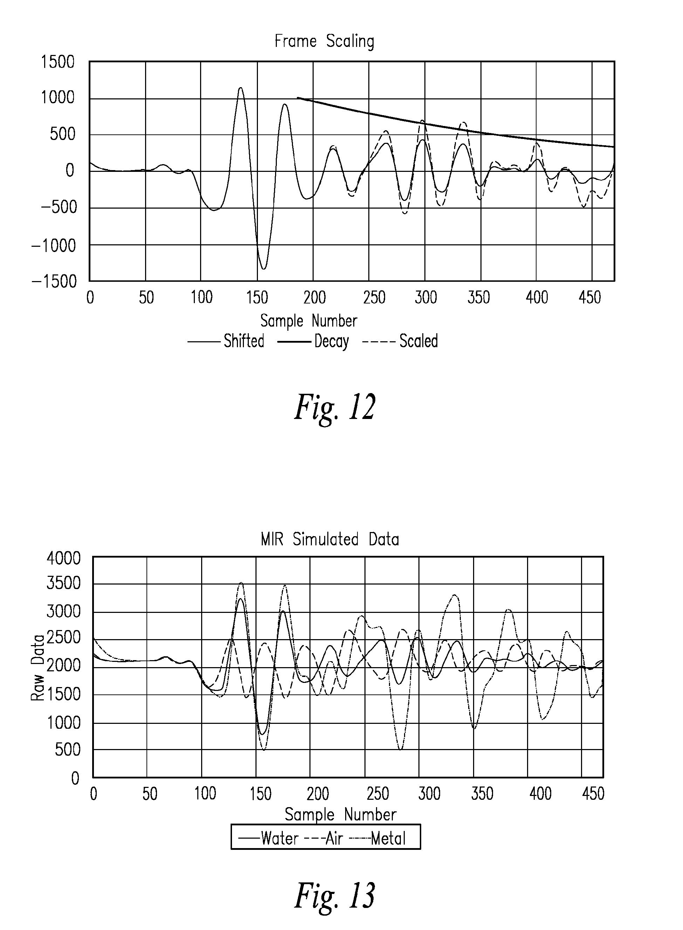

Calibration: Calibration can be performed by setting default constructor definitions in the processing source code. Suggested default values for these parameters are provided below. Alternatively, calibration can be performed with a phantom response. As an example, FIG. 12 shows a sample return (solid oscillating line) from a water phantom. The waveform has been set to zero mean. The default decay curve, F(t), is shown above the sample return. From the decay constant, scaling has been performed by 1/F(t), producing the dotted line. An efficient and relatively robust method to estimate the end of the precursor is to find the peak amplitude (value 1150 at sample 137), multiply by 0.4, draw a line, and search for line crossings (open circles). The fourth crossing will typically be located near the end of the precursor. Note: A dead zone of 4 points should be established in the zero crossing algorithm in order to minimize the possibility of noise contamination in the crossing count. Note: The factor of 0.4 is not particularly sensitive, as long as it is puts the threshold well above the noise preceding the precursor (anything in the range of 0.3 to 0.6 is acceptable). Likewise, four threshold crossings are sought. Two to four is acceptable, more being better; but do not exceed four. Once the precursor is identified, the decay factor, of the form a • exp(- k • t), can be derived from a curve fit to 1) the standard deviation of the first, say, 50 points after the precursor and 2) the standard deviation of the last 50 points of the frame. This is not a critical factor, as long as it is proportional to the power of the beginning of the "good" part of the frame with the end of the frame. A nominal factor of k = 0.004 approximates the behavior of typical MIR electronics.

Error Detection: Perform a preliminary examination of the data from all eight locations in order to screen for obvious electronic or operator error (bad scan, bad body contact, etc.). The easiest check is to obtain the eight locations of data and calculate the standard deviation. These measures will be representative of the overall departure of the data from the reference. If that departure is much more than that expected for a pneumothorax, then the scan will be declared in error. The example shown in FIG. 13 is for water, air, and metal. The standard deviation for the water scan is high through the precursor (about 35 up through sample 190) and low (about 12) throughout the rest of the frame. For air, the precursor standard deviation is considerably lower (about 19) and thus easily differentiated. For metal, the precursor is about the same as that for water, but the post-precursor standard deviation is considerably higher (about 39) and also easily differentiated. Note: For this measure, the mean is removed and scaling is not performed. Note: Rather than calculating the variances only about the precursor, it is useful to extend the dividing line somewhat to the right of the precursor (about 50 points). If the ratio of the precursor standard deviation of any of the eight locations to that of the water phantom is less than 0.7 (TBD) a low side scan error (air-like) should be declared. If the ratio of the post-precursor standard deviation to that of the water phantom is greater than 2.0 (TBD) a high side scan error (metal-like) should be declared. A metal-like error can occur when there is metal in the torso (e.g., shrapnel). Therefore, the pneumothorax detector can detect the presence of metal in the torso in the field of the scan (i.e., a scanning field), based on the received echoes of MIR signals. The pneumothorax detector can provide a scanning error indication if metal is present in the torso, and/or a further indication that metal is present.

The following list contains various example design parameter defaults for the pneumothorax detection method discussed above. The frame parameters (windowing and scaling) may be further refined by self-calibration, if desired. Pneumothorax detection and error detection may be refined by data processing of gathered data.

### Frames

| | |
|---|---|
| Locations | Use all 8 locations, $L_1, L_2, L_3, L_4, L_5, L_6, L_7, L_8$ |
| Frame Number | Use the first frame if windowing is present; the second otherwise |
| Frame Averaging | Do not average multiple frames; use one frame per location |
| Window Start | Sample 190; this typically avoids the precursor and is static |
| Window Stop | Sample 470, the last data point; do not truncate points |
| Scaling | 1.0 * exp(0.004 / (Sample Number- Window Start)) |
| Data Averaging | Remove the average within the window, after scaling |

### Pneumothorax Detection

| | |
|---|---|
| Reference Vector | Average the n selected reference frames, $R = \{r_1 ... r_n\}$ |
| Correlation | Obtain Pearson correlations $(R,L_3), (R,L_4), (R,L_7), (R,L_8)$ |
| Detection Threshold | Positive if any one correlation is less than 0.90 |

### Error Detection

| | |
|---|---|
| Test Vector | An internally stored water phantom with no windowing or scaling |
| Test StdDev | StdDev of zero mean Test Vector before and after precursor or the precursor extension |
| Max Threshold | Error if StdDev of post-precursor > 2.0 times water StdDev |
| Min Threshold | Error if StdDev of precursor < 0.7 times water StdDev |

A discussion of the circuitry for performing MIR pneumothorax detection is provided below. Background information about MIR can be found in U.S. patent number 5,361,070 to McEwan, incorporated herein by reference in its entirety. Conventional radar sends out short bursts of single-frequency (i.e., narrow-band) electromagnetic energy in the microwave frequency range to obtain information about a scene. Other radars step through multiple frequencies. An impulse, or ultrawide-band, radar such as MIR sends individual pulses that contain energy over a very wide band of frequencies (e.g., 800 MHz - 6 GHz). The shorter the pulse, the wider the band. Because the pulse is very short, little power (e.g., less than 1 Watt) is needed to generate the signal. MIR systems can generate and detect sub-nanosecond pulses.

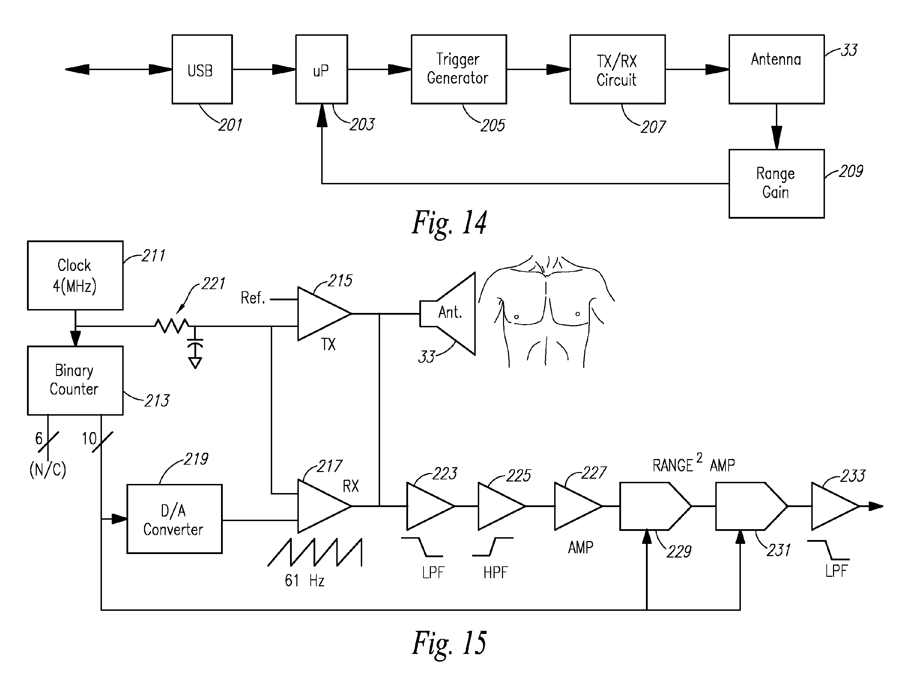

A top-level block diagram of the pneumothorax detector is provided at FIG. 14. The detector includes a USB portion 201 for communicating with a remote processor and charging the pneumothorax detector. A processor 203, such as a microcontroller or microprocessor, controls the operations pneumothorax detector and communicates with the remote processor. For example, the processor 203 controls the operations of a trigger generator 205. The trigger generator 205 controls the timing of the transmission and reception of MIR signals by the transmitting/receiving (TX/RX) circuitry 207. The MIR signals are transmitted and received through a wideband horn antenna 33, which is discussed in detail below. The detector further includes range gain circuitry 209. The range gain circuitry 209 provides a range-squared amplifier that compensates for the attenuating effects of distance on the received MIR signals.

A more detailed function block diagram is provided at FIG. 15. A packaged oscillator 211 generates a desired time base or clock (e.g., 4 MHz, 8 MHz, etc.) for use by a 16-bit binary counter 213, as well as transmit 215 and receive 217 circuits. A transmit pulse is triggered by comparing the 4 MHz clock signal to a constant reference voltage. Thus, the pulse repetition rate is a constant 4 MHz.

The transmit pulse is converted into a radar generating pulse by a step recovery diode (not shown). Another step recovery diode (not shown) is used to sample the receive signal.

The 16-bit binary counter 213 has outputs that are connected to a digital-to-analog (D/A) converter 219. By feeding the clock signal to the combination of a 16-bit binary counter 213 and D/A converter 219, a 61 Hz (4 MHz/65,536) sawtooth wave is generated as indicated in FIG. 15. The sawtooth wave forms a reference level for the receive trigger. The 6 least significant bits of the counter 213 are not connected to the D/A converter 219. By discarding these bits, each point on the ramp is sampled and averaged $2^6 = 64$ times, meaning that the receive signal is inherently low-pass filtered.

The receiver 217 is triggered when the 4 MHz clock crosses the threshold of the sawtooth. This has the effect of providing a precisely controlled delay in the receive pulse over the 16 ms (1/61) period of the sawtooth. Note that a small RC delay 221 is added to the trigger signal. This has the effect of slowing the transition time of the trigger to the point where the receive comparator can use the sawtooth wave to achieve the variable delay required.

The worst-case resolution of the receiver is obtained by dividing the period of the 4 MHz clock by $2^{10}$: 250 ns/1024 = 244 ps. This is improved in practice by adjusting the slope and offset of the sawtooth to achieve desired near and far distances for MIR data acquisition. Receive pulses, for example, can take place over a window of approximately 3.5 ns. This 3.5 ns window is achieved by the adjustment of the slope and offset of the sawtooth waveform. The temporal span of 3.5 ns corresponds to a spatial resolution of $(3 \times 10^8$ m/s x $3.5 \times 10^{-9}$ s)/1024 = 1 mm (free space).

The output of the receiver 217 is fed through low-pass 223 and high-pass 225 filters and an amplifier 227 before being fed through a pair of multiplying D/A converters 229, 231. The output of each of the D/A converters 229, 231 is the product of its analog input and the 10-bit binary amplitude of the ramp function (output from the binary counter 213). By cascading the D/A converters 229, 231 as shown, a range-squared amplifier is achieved, which compensates for the attenuating effects of distance on the received MIR signals. The output of the range-squared amplifier is fed through a low-pass filter 233 prior to digitization and application of the pneumothorax detection algorithm.

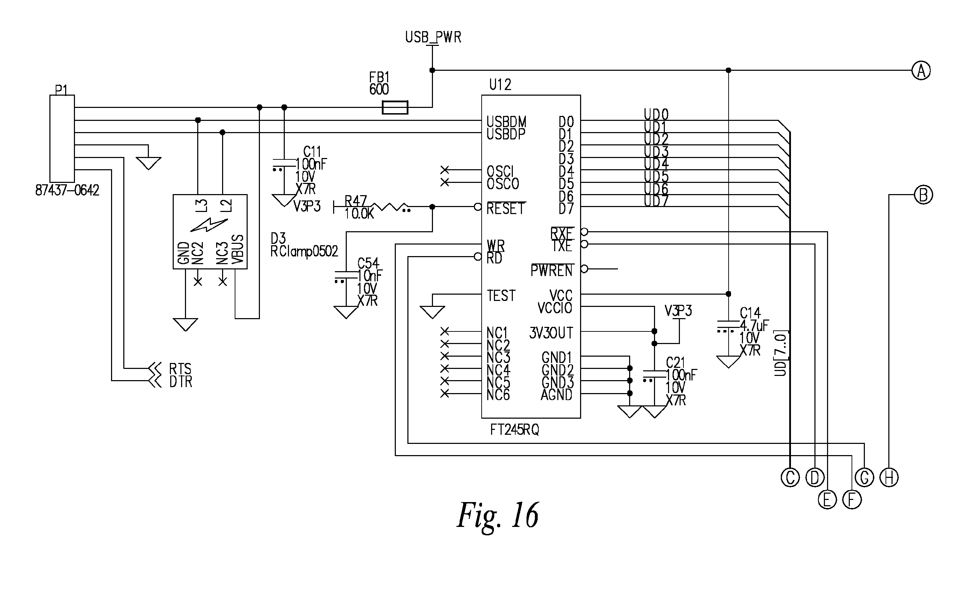

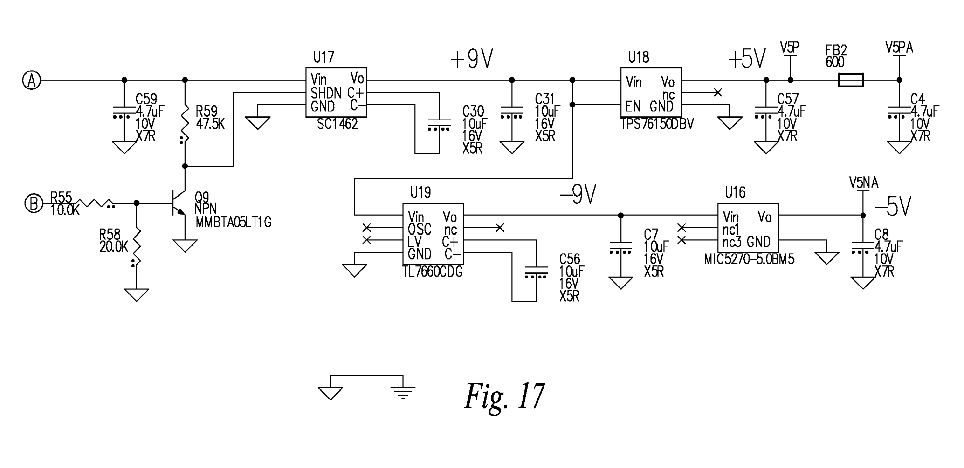

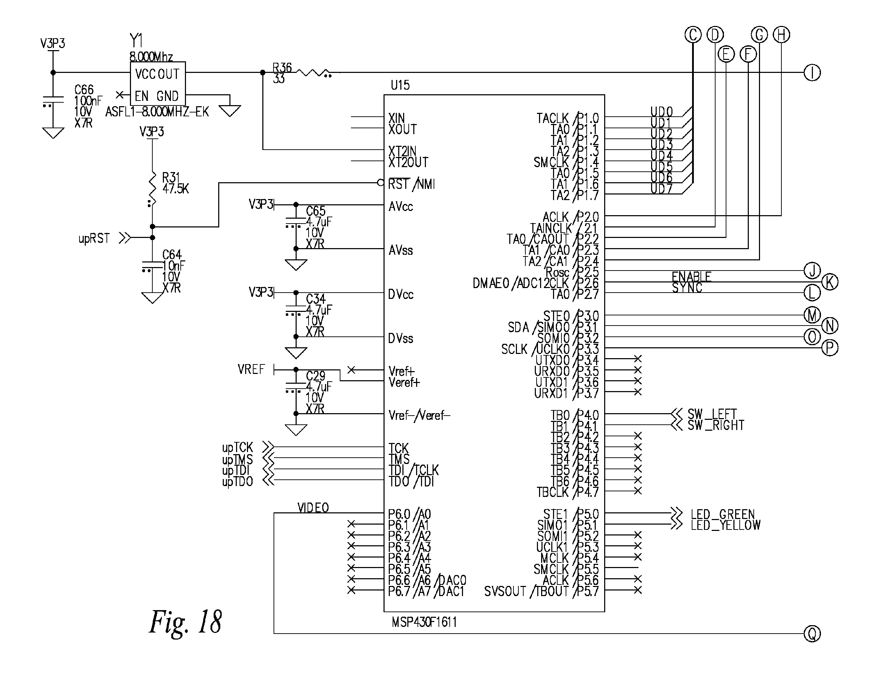

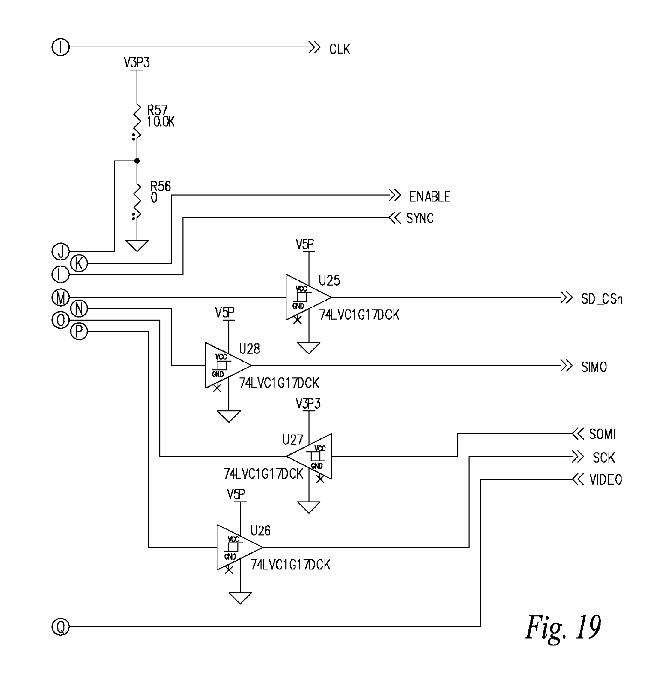

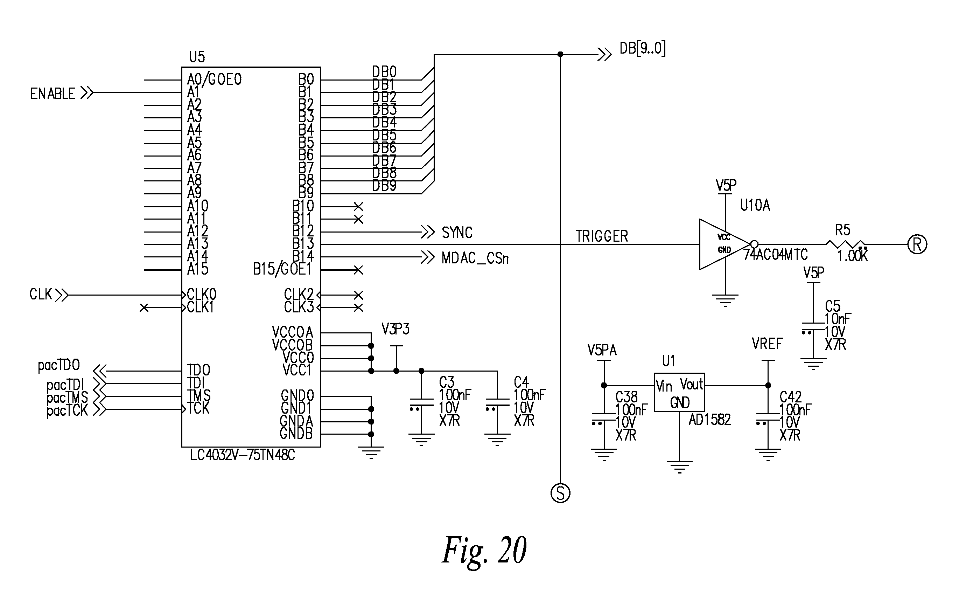

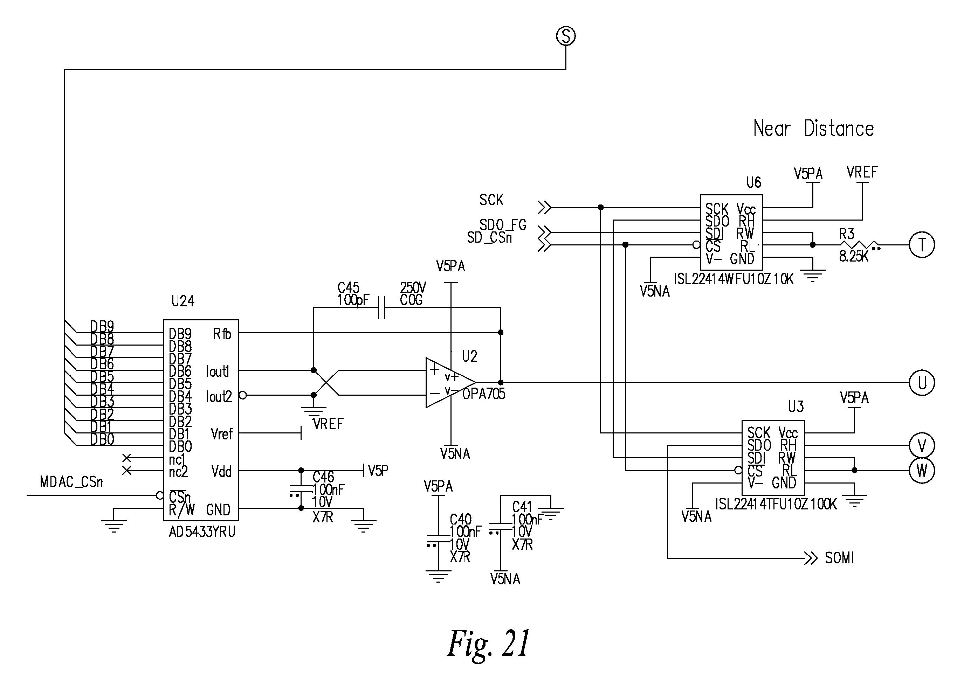

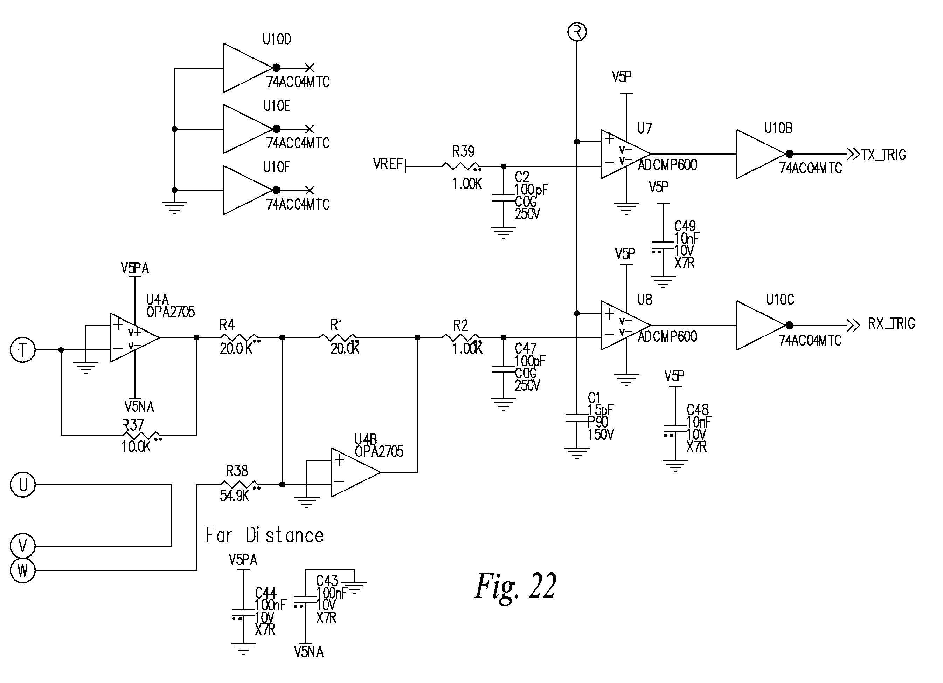

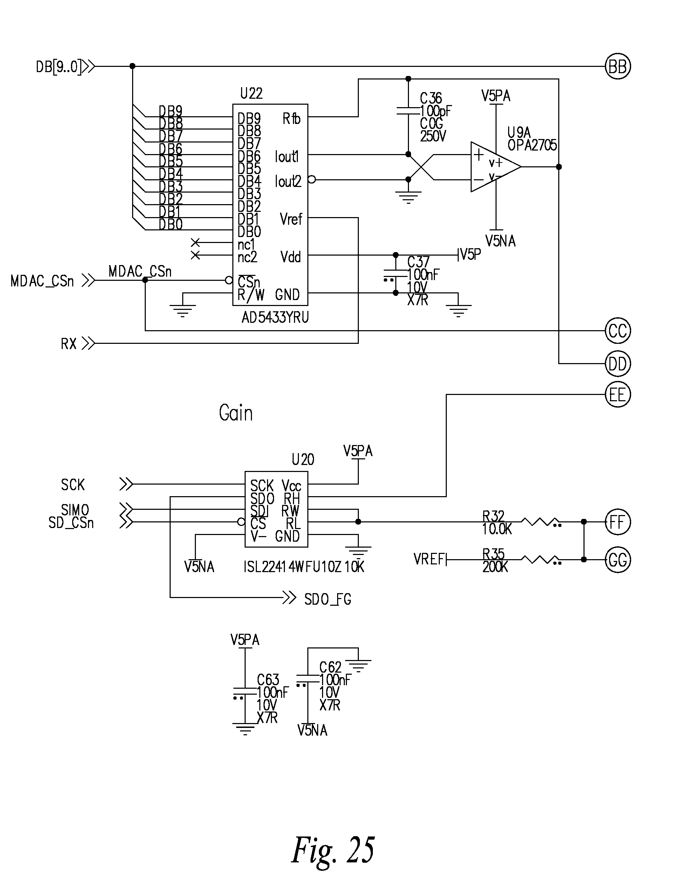

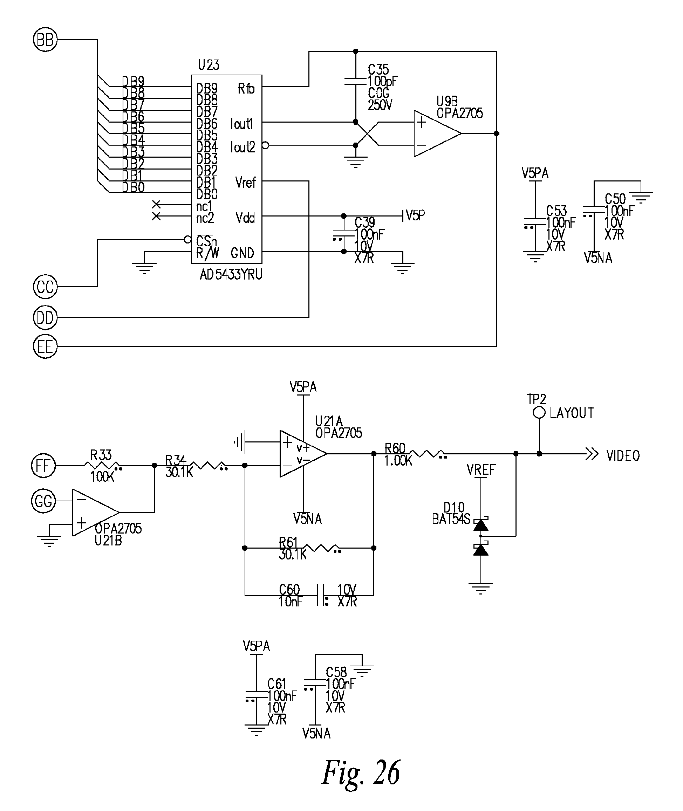

Turning to FIGS. 16-28, schematics of the circuitry shown in block diagram form in FIGS. 14 and 15 are provided. The circuitry can be provided on a single MIR circuit board. The embodiment shown in FIGS. 16-28 is intended to be interfaced to and powered from the USB port of a PDA-type host device. However, it is to be appreciated that the circuitry can also be part of a self-contained pneumothorax detector (e.g., as shown in FIG. 1).

In an embodiment, the MIR circuitry generates a series of 65,535 sub-nanosecond impulses to the antenna. Analog hardware constructs an apparent single-pulse return signal (termed video herein) by taking 64 samples of the return signal at each of 1024 intervals over 16.384ms. A wideband horn generates the waveform and the radiated energy only occurs in one direction. In an embodiment, the MIR circuitry has the capability of measuring reflections out to approximately 6 inches (15 cm).

With reference to FIGS. 16-22, an 8-MHz oscillator is divided down to 4 MHz to drive the timing circuits. The time base is generated by dividing this 4 MHz clock by 65,536 (64 samples x 1024 intervals.) The 1024 represents the 10 most significant bits of the binary counter U5 ($2^{10} = 1024$). A square-wave is generated with a period of 16.384ms (every 65,536 counts.) The falling edge of this signal triggers the start of the video sweep. The constructed video signal is captured by the microprocessor and analyzed by the microprocessor and/or a controlling PDA.

All power is derived from the 5V USB supply. Although the USB supply is nominally +5V, in reality it has a wide potential range of 4.40V minimum to 5.25V maximum. To provide a more reliable voltage source, this voltage is doubled and then re-regulated with linear regulators. U17 doubles the USB voltage to approximately +9V. U18 then provides a clean/stable +5V (V5P) to drive the onboard logic. This +5V is further noise isolated to separate the digital power from the analog 5V (V5PA) components via FB2. U19 inverts the +9V to -9V. U16 generates a clean analog -5V (V5NA) for use by the analog circuits. U12 generates +3.3V (V3P3) for use by the microprocessor and the complex programmable logic device (CPLD) U5. Average power consumption of the MIR PCB assembly is less than 1W. The average power to the MIR PCB is limited by the USB 1W output power.

U12 provides a USB 2.0 compliant device interface for the MIR board. The interface connects to the microprocessor U15 via an 8-bit bus. This USB interface uses an appropriate cable to connect to an MC7X or any other USB 2.0 compliant host.

The microprocessor U15 stores the calibration setting, initiates and captures the resulting reflection signal, and communicates with the host PDA via the USB chip U12. All MIR-frequency RF energy transmission is controlled by the microprocessor.

Timing controls are derived from the 4 MHz clock U5. The square-wave clock signal (TRIGGER) is low-pass filtered by R5 and C1 to produce exponential edges with a 15 nsec time-constant and amplitude of 5V. This exponential ramp drives the non-inverting inputs of two ultra-fast comparators, U7 & U8. The inverting input of U7 is held at +2.5V from reference U1. This causes U7 to switch by a fixed delay following each clock edge (RC delay plus propagation delay ~13 nsec). A rising edge at the output of U7 triggers the transmitter output impulse. The inverting input of U8 is driven with a DC voltage plus a linear voltage ramp. The DC component is adjusted to delay the switching of U8 with respect to U7 by the time interval corresponding to the MIR near range (i.e. time delay between sending out a transmit impulse and receiving a reflection at minimum target distance). The AC component sweeps this delay between the MIR near distance and the MIR far distance (i.e. time delay between sending out a transmit impulse and receiving a reflection from the maximum target distance). A rising edge at the output of U8 triggers a sampling pulse at the receiver input.

The real time duration of the near-to-far range sweep is the video timebase and is around 16.38 msec. The time base is generated by binary counter in U5. It divides the 4 MHz clock by 65,536 to generate the time base. It is a square-wave and appears at pin 38 of U5, where it is labeled SYNC. A falling edge on this line corresponds to the start of a video sweep.

The 10 most significant bits of the binary counter drive a current-output multiplying D/A converter (DAC) U24. Coupled with a +2.5-V reference input and a current-to-voltage converter (U2), the DAC produces a triangle-wave that ramps linearly from 0.0v to -2.5v over the duration of the timebase.

Op amp U4B sums the AC and DC terms that sweep the receive trigger delay. The AC term (Far Distance) is obtained from U3 and is adjusted with potentiometer U3. The DC term (Near Distance) is generated by U4A and is adjusted with potentiometer U6. On a calibrated MIR board, the delay between the transmit-impulse and the receive-sample pulse sweeps from around 1 nsec to 4 nsec.

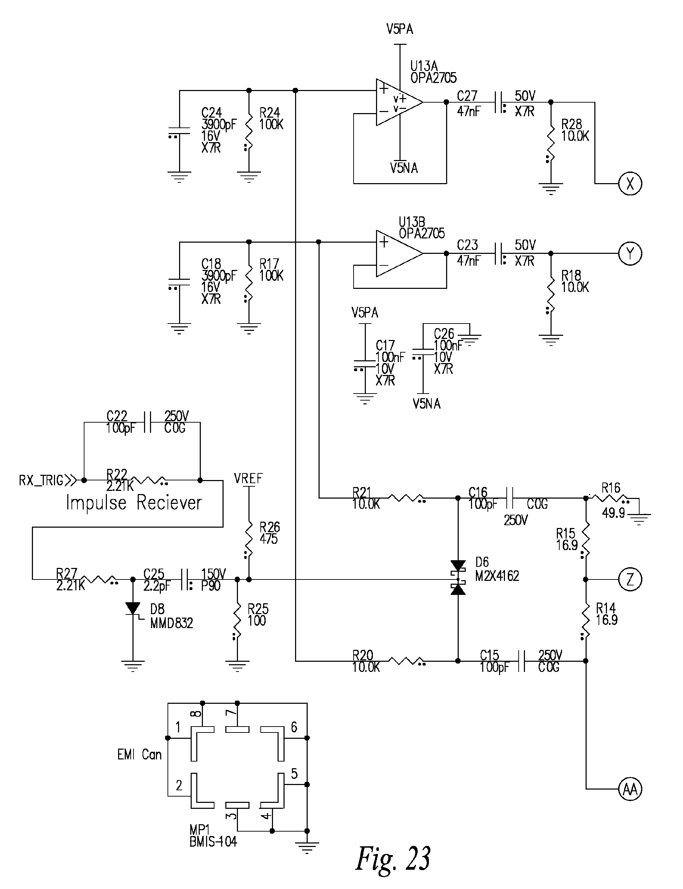

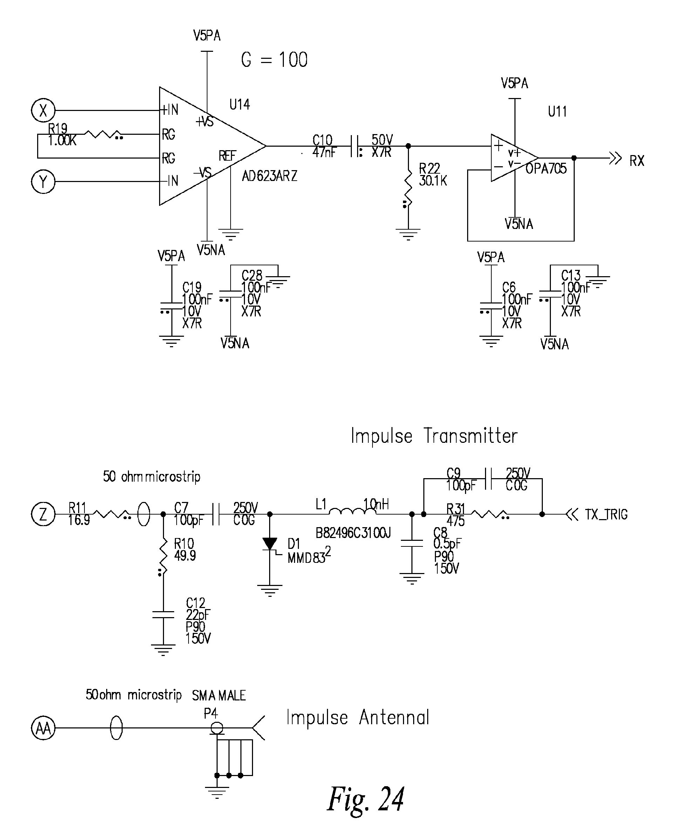

Circuitry for transmitting, receiving and processing (e.g., range-squared amplifying) the MIR pulses is shown in FIGS. 23-26. All of the components shown on FIGS. 23 and 24 can be located on the MIR board under an electromagnetic interference (EMI) shield can. The MIR functions by transmitting a sub-nanosecond impulse and constructing the time-domain reflection by under-sampling the return waveform. Between the near and far distances (plus the return path) there are 65,536 samples, comprising 64 samples taken at each of 1024 evenly spaced intervals over 16.384 ms. Distance resolution of the MIR is limited by system bandwidth rather than the number of samples. The large sample count instead enhances the signal-to-noise ratio by analog averaging at each distance.

The transmitter and receiver share a common antenna. The subcircuit comprising C15, C16, R11, R14, R15 and R16 connect these three sub-systems. This subcircuit optimizes loading of both the transmitter and the antenna, thereby minimizing signal reflections and ringing. In an embodiment, the transmitter output impedance is 50Ω (see Equation 1), and the transmitter drives a load resistance of 50Ω (see Equation 2). Likewise the 50Ω MIR antenna drives a 50Ω load. This matching helps optimize the receiver's ability to detect low-level external reflection signals.

## Equation 1 TX load

$$TX = R14 + (R15 + R16) \parallel (R14 + Rant) = 50\Omega \text{ load.}$$

## Equation 2 ANT load

$$\text{Ant load} = R14 + (R11 + R10) \parallel (R15 + R16) = 50\Omega \text{ load}$$

A transmit impulse is initiated when U7 switches high. This causes the output of inverter U10B to go low, turning off the forward current flowing through D1. Reverse current flows briefly in D1, but being a step recovery or "snap" diode, D1 then turns off very abruptly (~70 psec). When this happens, the associated pulse forming network (components between U10B output and R11) generate a fast negative-going impulse (~1 volt at P4). With an MIR antenna connected to P4, a second antenna can be used to observe the transmitted near field using a high-speed oscilloscope (e.g. Tek DSA70604). With 1" antenna separation the transmit pulse appears as a monocycle with a period of ~500 psec and a peak-to-peak amplitude of ~50 mV.

Receiver sampling is initiated when U8 switches high. Snap diode D8 delivers a -1 volt, 1 nsec pulse to capacitor C25. The pulse couples though C25, turns on each diode of D6, and delivers a current of ~4 mA (Icharge) to each C15 and C16. The current flows for approximated 70 psec (Tpulse) resulting in a charge increment of 280fC (ΔC) being delivered to each C15 and C16. With a 4-MHz sampling rate (Fsample) the average charging current (Ic) through C15 and C16 is 1.1 μA. These capacitors discharge continuously through R17 and R24 respectively, generating a nominal offset of 112 mV (Voffset) at C18 and C24. These voltages are a good indication of proper function of the sampling receiver. (see Equation 3, Equation 4, Equation 5).

## Equation 3 ΔC

$$\Delta C = I_{charge} * T_{pulse} = 4 \text{ mA} * 70 \text{ ps} = 280 \text{ fC}$$

## Equation 4 Ic

$$Ic = \Delta C * F_{smaple} = 280\ fC * 4\ MHz = 1.12\ uA$$

## Equation 5 Voffset

$$V_{offset} = Ic * R24 = 1.12\ uA * 100k = 112\ mV$$

If no transmit pulses were present the voltages on C18 and C24 would be DC only. Since these voltages are high-pass filtered by C27 & C23, there would be no video output from the MIR board.

If transmit pulses were present but the antenna was a replaced with an ideal 50Ω load, identical (and attenuated) transmit impulses would be present at C16 and C15. The voltages at C18 and C24 would reconstruct the transmit waveform. However they would be similar waveforms and the difference value would be zero coming out of the instrumentation amplifier U14. Again, no video output from the MIR board.

When an MIR antenna is pulsed, reflections are returned to C15 and C16. With an ideal antenna these reflections would come from external targets only. With an actual MIR antenna there are also some reflections from within. The return signals connect to C15 directly while the C16 signal is attenuated by 6dB by R10, R16, R15, R14 and R11. In other words the receive signal appears as a differential voltage at C16 and C15. When the sampling diode D6 turns on, the pump action of C25 no longer diverts equal charges into C16 & C15. The capacitor with a more positive common-mode voltage will take a larger share of charge, and in turn create a differential AC voltage at C18 and C24. This voltage is amplified, band-pass filtered, and range-square corrected to become the MIR video output.

The power density (watts/meter$^2$) of a far field electromagnetic wave attenuates with the inverse square of distance. The same attenuation occurs on the return path from a reflecting target, resulting in an inverse forth-power relationship. Since the receiver responds to the voltage field, its signals are attenuated by the inverse square of antenna-to-target distance. To correct for this loss, a pair of cascaded multiplying DACs (U22, U23) are used to boost the receive signal. Each DAC functions as an amplifier that ramps up gain linearly with time using the same digital controls as the triangle-wave (sawtooth) generator described above. Between them, the DACs provide the range-square correction function.

Op amp U21 B combines the range-corrected video signal together with a DC voltage to provide a video baseline of +1.25v. Signal gain is adjusted by digital pot U20 to keep the video output voltage within the range of 0.0v to +2.5v.

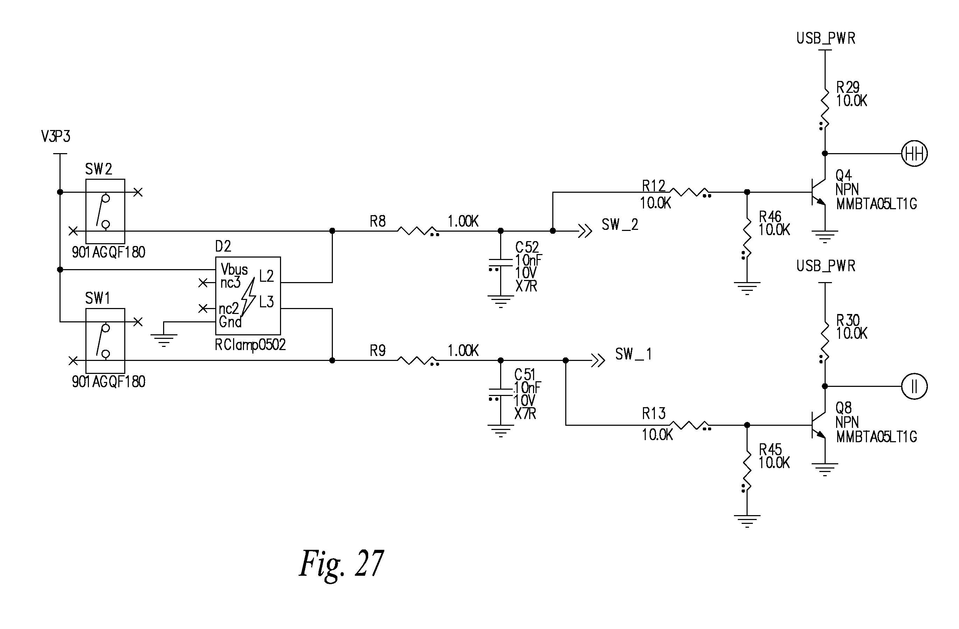

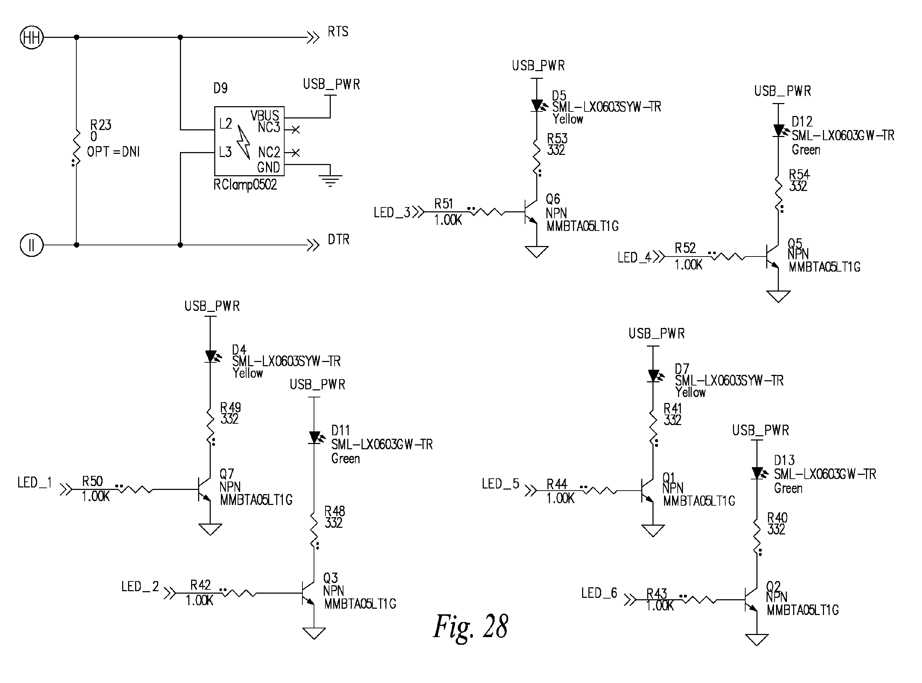

User interface elements are shown in FIGS. 27 and 28. Two push buttons are implemented on the left (e.g., left scan button 21 in FIG. 1) and right side of the handheld unit. These buttons are used to initiate and sequence through the test procedure. SW1 & SW2 are elecrostatic discharge (ESD) protected by D2, buffered by Q4 and Q8, and passed on to the microcontroller. BiColor LEDs D4, D5 and D7 provide direct user feedback. Each LED is controlled by a pin from the microprocessor.

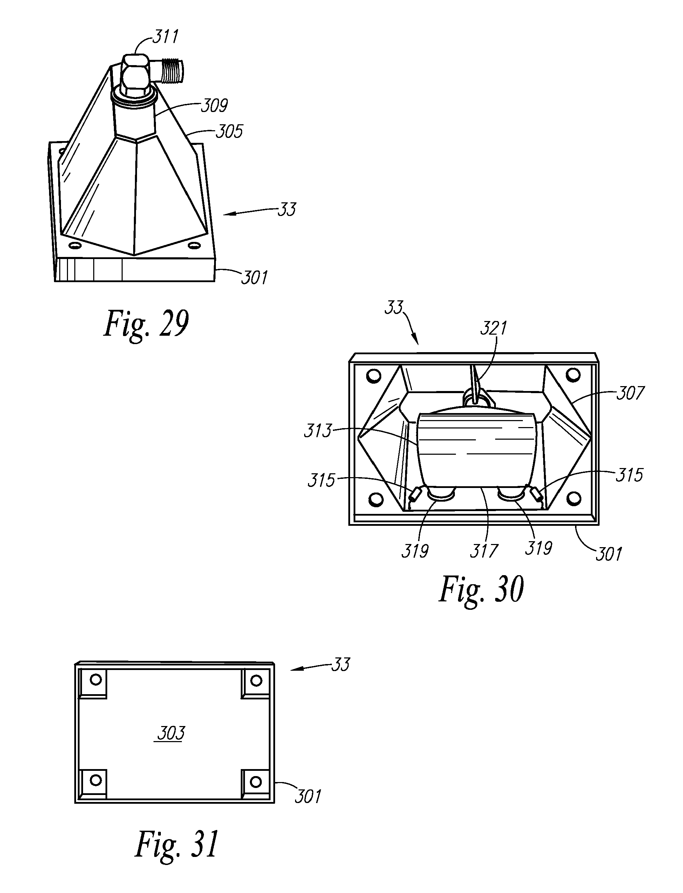

Turning to FIGS. 29-31, a wideband horn antenna 33 for the pneumothorax detector is shown in detail. Through the horn antenna 33, MIR signals are transmitted by the pneumothorax detector into the torso of a patient and corresponding echoes are received.

Horn antennas have previously been used in MIR applications. For example, U.S. patent number 6,348,898 to Rosenbury et al., incorporated herein by reference in its entirety, describes a wideband horn antenna for use in MIR applications.

The horn antenna 33 shown in FIGS. 29-31 includes a hollow base 301 and a radome 303 located within the base 301. The radome is fabricated from a low loss dielectric material such that reflections from the antenna-body interface are minimized when the pneumothorax detector is in use. The radome 303 provides impedance matching between the skin of the patient and the pneumothorax detector. Example materials of construction for the radome 303 include low loss plastics (e.g., ECCOSTOCK® HiK available from Emerson & Cuming Microwave Products), ceramics, glasses (e.g., soda lime based glasses), titanium dioxide, and polyethylene terephthalate (PET). Within the housing of the pneumothorax detector, the antenna 33 is located above the patient contact area 15 (see FIG. 1), and the radome 303 is in physical contact with the housing in the patient contact area.

As shown in FIG. 29, the horn antenna 33 includes a horn 305 that extends away from the base 301. The horn 305 has the shape of an irregular hexagon. FIG. 30 provides a bottom perspective view of the antenna 33 with the radome removed. It can be seen in FIG. 30 that the horn forms an inner cavity (i.e., a radiation cavity) that ends at a large antenna aperture 307 into the base 301. The radome 303, when installed within the base 301, covers the antenna aperture 307.

The horn has an upper closed end 309 and a connector 311 that penetrates the closed end. The connector 311 is shown as a right angle connector, however, the connector could also be a straight connector. The connector 311 connects the horn antenna 33 to the MIR board 31 (see FIG. 3). In an embodiment, the connector 311 is a coaxial SMA connector with a characteristic impedance of 50 ohms.

The center conductor of the connector 311 carries MIR pulses to a radiator 313 located with the radiation cavity formed by the horn 305. The radiator 313 has the shape of a curved fin and has a taper toward the connector 311. MIR pulses are transmitted from the radiator 313 and through the radome 303 into a patient's torso. The radiator 313 is electrically coupled to the horn 305 through termination resistors 315. The termination resistors 315 minimize low frequency reflections back to the receiver on the MIR board. The termination resistors 315 can be impedance matched to the transmission line feeding the antenna 33 (e.g., 50 ohms). A lip 317 of the radiator 313 rests on insulators 319 that are attached to the horn 305.

Sidewalls of the horn antenna 33 extend from the antenna aperture 307 to the closed end 309. An internal ridge 321 projects into the radiation cavity from one of the sidewalls. The internal ridge 321 is arc-shaped. The presence of the internal ridge 321 affects the impedance of the antenna.

It should be evident that this disclosure is by way of example and that various changes may be made by adding, modifying or eliminating details without departing from the fair scope of the teaching contained in this disclosure. The invention is therefore not limited to particular details of this disclosure except to the extent that the following claims are necessarily so limited.

**Claims**

1. A method of deriving pneumothorax information from a body, based on reflected MIR signals, received, using a micropower impulse radar (MIR) scanning device, from first and second right torso locations of said body and from first and second left torso locations of said body;

    - comparing at least one of the reflected MIR signals to self-referenced reference data; and
    - generating an output based on a result of the step of comparing at least one of the reflected MIR signals to the self-referenced reference data.

2. The method of claim 1, wherein the step of comparing at least one of the reflected MIR signals to self-referenced reference data is performed by a handheld MIR scanning device, or by a remote processor.

3. The method as in any one of claims 1 or 2, wherein one of the first right torso location and the second right torso location is substantially on a right anterior axillary line of the body and the other one of the first right torso location and the second right torso location is substantially on a right midclavicular line of the body, and wherein one of the first left torso location and the second left torso location is substantially on a left anterior axillary line of the body and the other one of the first left torso location and the second left torso location is substantially on a left midclavicular line of the body.

4. The method as in any one of claims 1 or 2, further comprising, based on reflected MIR signals, received, using a micropower impulse radar (MIR) scanning device, from third and fourth right torso locations of said body and from third and fourth left torso locations of said body:

    determining the presence of metal in a scanning field based on the result of the step of comparing at least one of the reflected MIR signals to the self-referenced reference data; and
    determining whether a scanning error has occurred based on the result of the step of comparing at least one of the reflected MIR signals to the self-referenced reference data,
    wherein both of the first right torso location and the second right torso location are substantially on a right anterior axillary line of the body,
    wherein both of the third right torso location and the fourth right torso location are substantially on a right midclavicular line of the body,
    wherein both of the first left torso location of the body and the second left torso location of the body are substantially on a left anterior axillary line of the body, and
    wherein both of third left torso location and the fourth left torso location are substantially on a left midclavicular line of the body.

5. The method as in any one of claims 1-4, further comprising the steps of:

determining self-referenced reference data;
wherein the step comparing at least one of the reflected MIR signals to the self-referenced reference data includes statistically correlating the at least one of the reflected MIR signals to the self-referenced reference data.

6. The method of claim 5, wherein the step of determining the self-referenced reference data includes cross-correlating scanning results.

7. A micropower impulse radar (MIR) pneumothorax detector, comprising:

a processor; and
a handheld MIR scanner, comprising:

a device housing;
an antenna located within the device housing;
circuitry that generates MIR signals; and
circuitry that processes received echoes of the MIR signals and provides the processed received echoes to the processor, wherein the processor to process reflected MIR signals, received from first and second right torso locations of a body and from first and second left torso locations of said body; to compare at least one of the reflected MIR signals to self-referenced reference data; and to generate an output based on a result of the step of comparing at least one of the reflected MIR signals to the self-referenced reference data.

8. The detector of claim 7, wherein the step of comparing at least one of the reflected MIR signals to self-referenced reference data is performed by the handheld MIR scanner, or by a remote processor.

9. The detector in any one of claims 7 or 8, wherein one of the first right torso location and the second right torso location is substantially on a right anterior axillary line of the body and the other one of the first right torso location and the second right torso location is substantially on a right midclavicular line of the body, and
wherein one of the first left torso location and the second left torso location is substantially on a left anterior axillary line of the body and the other one of the first left torso location and the second left torso location is substantially on a left midclavicular line of the body.

10. The detector as in any one of claims 7 or 8, wherein based on reflected MIR signals received from third and fourth right torso locations of said body and from third and fourth left torso locations of said body, the processor to
determine the presence of metal in a scanning field based on the result of the step of comparing at least one of the reflected MIR signals to the self-referenced reference data; and
determine whether a scanning error has occurred based on the result of the step of comparing at least one of the reflected MIR signals to the self-referenced reference data,
wherein both of the first right torso location and the second right torso location are substantially on a right anterior axillary line of the body,
wherein both of the third right torso location and the fourth right torso location are substantially on a right midclavicular line of the body,
wherein both of the first left torso location of the body and the second left torso location of the body are substantially on a left anterior axillary line of the body, and
wherein both of third left torso location and the fourth left torso location are substantially on a left midclavicular line of the body.

11. The detector as in any one of claims 7-10, wherein the processor is further configured to:

determine self-referenced reference data;
wherein the step comparing at least one of the reflected MIR signals to the self-referenced reference data includes statistically correlating the at least one of the reflected MIR signals to the self-referenced reference data.

12. The detector of claim 11, wherein the self-referenced reference data includes cross-correlating scanning results.

**Fig. 1**

**Fig. 2**

Fig. 3

Fig. 4

Fig. 5

*Fig. 6*

*Fig. 7*

*Fig. 8*

Fig. 9

*Fig. 10*

*Fig. 11*

Frame Scaling

*Fig. 12*

MIR Simulated Data

*Fig. 13*

*Fig. 14*

*Fig. 15*

EP 2 698 110 A1

*Fig. 16*

*Fig. 17*

*Fig. 18*

*Fig. 19*

*Fig. 20*

Near Distance

*Fig. 21*

**U10D**
74AC04MTC

**U10E**
74AC04MTC

**U10F**
74AC04MTC

V5P

**R39**
1.00K

VREF

**C2**
100pF
COG
250V

**U7**
ADCMP600

v+
v−

V5P

**C49**
10nF
10V
X7R

**U10B**
74AC04MTC

TX_TRIG

V5PA

**U4A**
OPA2705

v+
v−

V5NA

**R37**
10.0K

T

**R4**
20.0K

**R1**
20.0K

**R2**
1.00K

**C47**
100pF
COG
250V

**C1**
15pF
P90
150V

V5P

**U8**
ADCMP600

v+
v−

V5P

**C48**
10nF
10V
X7R

**U10C**
74AC04MTC

RX_TRIG

**U4B**
OPA2705

**R38**
54.9K

U

V

W

Far Distance

V5PA

**C44**
100nF
10V
X7R

**C43**
100nF
10V
X7R

V5NA

*Fig. 22*

*Fig. 23*

G = 100

V5PA

X

R19
1.00K

Y

+IN
+VS    U14
RG
RG
-IN   -VS   REF

AD623ARZ

C10
47nF

50V
X7R

R22
30.1K

V5PA

U11

+  v+
-  v-   OPA705

V5NA

RX

V5NA

V5PA

C19
100nF
10V
X7R

C28
100nF
10V
X7R

V5NA

V5PA

C6
100nF
10V
X7R

C13
100nF
10V
X7R

V5NA

Impulse Transmitter

50 ohm microstrip

Z    R11
16.9

C7
100pF

250V
C0G

R10
49.9

D1
MMD83[2]

L1    10nH

B82496C3100J

C9
100pF

250V
C0G

R31
475

TX_TRIG

C8
0.5pF
P90
150V

C12
22pF
P90
150V

50 ohm microstrip  SMA MALE

AA

P4

Impulse Antennal

*Fig. 24*

*Fig. 25*

*Fig. 26*

*Fig. 27*

Fig. 28

*Fig. 29*

*Fig. 30*

*Fig. 31*

**Europäisches Patentamt**
**European Patent Office**
**Office européen des brevets**

# EUROPEAN SEARCH REPORT

**Application Number**

EP 13 19 0157

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | "Noninvasive Pneumothorax Detector", INTERNET CITATION, 10 July 2007 (2007-07-10), XP007912893, Retrieved from the Internet: URL:http://www.medgadget.com/archives/2007/07/noninvasive_pneumothorax_detector.html [retrieved on 2010-04-20] * the whole document * ----- | 1-12 | INV. A61B5/113 |

TECHNICAL FIELDS SEARCHED (IPC)

A61B

The present search report has been drawn up for all claims

| Place of search | Date of completion of the search | Examiner |
|---|---|---|
| Munich | 20 December 2013 | Fischer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61155992 A
- US 61155997 A
- US 61156104 A

- US 5361070 A, McEwan
- US 6348898 B